# EUROPEAN PATENT APPLICATION

(11) **EP 1 607 101 A1**
(43) Date of publication of application: **21.12.2005**
(21) Application number: 04714023.1
(22) Date of filing: 24.02.2004
(51) Int. Cl.: A61K 45/00, A61K 38/17, A61P 1/16, A61P 31/12, A61P 35/00

(54) **DEGRADATION INHIBITOR FOR HEPATITIS B VIRUS X INTERACTING PROTEIN**

(30) Priority: 24.02.2003 JP 2003045711
(71) Applicant: DAIICHI PHARMACEUTICAL CO., LTD., Chuo-ku, Tokyo 103-8234 (JP)
(72) Inventor: DOI, Hirofumi, c/o Celestar Lexico-Sciences, inc., Chiba-shi, Chiba 2618501 (JP); MASUDA, Shoichi, C/o Celestar Lexico-Sciences, inc, Chiba-shi, Chiba 2618501 (JP); ISUMI, Yoshitaka, C/o Daiichi Pharma. Co., Ltd., Tokyo 134-8630 (JP); SAKAIYA, Noriko, c/o Daiichi Pharma. Co., Ltd., Tokyo 134-8630 (JP)
(74) Representative: De Clercq, Ann G. Y.
(86) International application number: PCT/JP2004/002138
(87) International publication number: WO 2004/073742

(57) **Abstract**

It was discovered that hepatitis B virus x interacting protein (XIP) is cleaved and degraded by caspase-1, and the cleavage recognition site of XIP was also discovered. Based on these findings, the present invention provides a method and composition for inhibiting the degradation of XIP, more particularly, inhibiting the degradation of XIP caused by caspase-1, preventing and/or treating a disease attributable to the degradation of XIP, inhibiting the replication and/or transcription of hepatitis B virus, preventing and/or treating hepatitis B, and preventing and/or treating hepatic cancer caused by hepatitis B virus.

## Description

### TECHNICAL FIELD

The present invention relates to a means for inhibiting the degradation of hepatitis B virus x interacting protein (hereinafter, may be abbreviated as XIP), more particularly, inhibiting the degradation of XIP caused by caspase-1, preventing and/or treating a disease attributable to the degradation of XIP, inhibiting the replication and/or transcription of hepatitis B virus (hereinafter, may be abbreviated as HBV), preventing and/or treating hepatitis B, and preventing and/or treating hepatic cancer caused by HBV.

### BACKGROUND OF INVENTION

Caspase-1 is a cysteine protease that is broadly distributed in the tissue and participates in the production of inflammatory cytokines and the induction of apoptosis. Within the cytoplasm, it exists in an inactive form (pro-form) and is converted to an active form by intracellular and extracellular stimuli. Recently, a correlation between hepatitis D virus infection and the amount of caspase-1 expression has been reported (Non-patent document 1).

Hepatitis viruses that cause hepatitis are classified into five genome types that are A type, B type, C type, D type and E type. Recently, the existence of F type and G type has been also acknowledged.

Hepatitis B virus causes acute and chronic hepatitis (Non-patent document 2). Furthermore, it is known that chronic hepatitis B caused by chronic HBV infection may lead to hepatocellular carcinoma (Non-patent document 3). Hepatocellular carcinoma is a cancer that is most frequently found among primary hepatic cancers and makes up about 90% of hepatic cancers. A protein derived from the HBV gene, such as, for example, hepatitis B virus x protein (hereinafter, may be abbreviated as HBx), has been reported to participate in the mechanism for the development of hepatocellular carcinoma from hepatitis B.

HBx, which lacks a DNA binding region, interacts with a transcription factor to work as a transactivator for various viral and cellular promoters (Non-patent document 4). Specifically, HBx promotes the transcription activity of these promoters. Particularly, it has been reported that HBx enhances the expression of the genes derived from HBV and promotes the replication of HBV (Non-patent document 5). Moreover, it is known that HBx activates an intracellular signal transduction pathway that plays a role in cell proliferation. In this way, it is considered that HBx is involved in carcinogenesis of the liver by its function, such as the transcriptional regulation of genes (Non-patent document 4).

On the other hand, XIP, which has been found from a cDNA library derived from hepatocellular carcinoma as a binding factor for HBx, has been reported to suppress the promoting effect of HBx on HBV replication (Non-patent document 6).

Documents referred to in this specification are listed hereunder:
Non-Patent Reference 1: Jiang Y et al., Zhonghua Gan Zang Bing Za Zhi., 2001, Vol.9, Special Edition: p. 9-11.
Non-Patent Reference 2: Seeger C. et al., Microbiology and Molecular Biology Reviews, 2000, Vol.64, p.51-68.
Non-Patent Reference 3: Beasley RP. et al., Lancet, 1981, Vol.2, p.1129-1133.
Non-Patent Reference 4: Rabe C. et al., Digestive Diseases, 2001, Vol.19, p.279-287.
Non-Patent Reference 5: Zhenming Xu TS et al., Journal of Virology, 2002, Vol.76, p.2579-2584.
Non-Patent Reference 6: Melegari M. et al., Journal of Virology, 1998, Vol.72, p.1737-1743.
Non-Patent Reference 7: Ulmer, Science, 1983, Vol.219, p.666-671.
Non-Patent Reference 8: "PEPUTIDO GOUSEI" (Japan), Maruzen Co., Ltd., 1975.
Non-Patent Reference 9: "Peptide Synthesis" (USA), Interscience, 1996.

### DISCLOSURE OF THE INVENTION

In the present invention, the present inventors carried out various studies to find a protein that interacts with caspase-1, for the purpose of providing a means for preventing and/or treating a disease attributable to the degradation of the proteins by caspase-1.

As a result, the present inventors predicted *in-silico* that caspase-1 interacts with XIP. Further, the present inventors experimentally demonstrated that XIP is cleaved and degraded by caspase-1, and found out the cleavage sites of XIP by caspase-1, to thus complete the present invention.

That is, one aspect of the present invention relates to an agent for preventing and/or treating a disease attributable to the degradation of hepatitis B virus x interacting protein (XIP), wherein the agent inhibits the cleavage of hepatitis B virus x interacting protein (XIP) by caspase-1.

Another aspect of the present invention relates to an agent for inhibiting the replication and/or transcription of hepatitis B virus (HBV), wherein the agent inhibits the cleavage of hepatitis B virus x interacting protein (XIP) by caspase-1.

A further aspect of the present invention relates to an anti-hepatitis B virus (HBV) agent, wherein the agent inhibits the cleavage of hepatitis B virus x interacting protein (XIP) by caspase-1.

A further aspect of the present invention relates to an agent for preventing and/or treating hepatitis B, wherein the agent inhibits the cleavage of hepatitis B virus x interacting protein (XIP) by caspase-1.

A still fiuther aspect of the present invention relates to an agent for preventing and/or treating hepatic cancer, wherein the agent inhibits the cleavage of hepatitis B virus x interacting protein (XIP) by caspase-1.

A further aspect of the present invention relates to a peptide consisting of an amino acid sequence set forth in SEQ ID NO: 2, 3, 4, 5, 6, or 7 in the sequence listing.

A further aspect of the present invention relates to a peptide comprising an amino acid sequence set forth in SEQ ID NO: 2, 3, 4, 5, 6, or 7 in the sequence listing.

A further aspect of the present invention relates to an agent for inhibiting the degradation of hepatitis B virus x interacting protein (XIP), wherein the agent comprises the aforementioned peptide.

A still further aspect of the present invention relates to an agent for inhibiting the degradation of hepatitis B virus x interacting protein (XIP), wherein the agent comprises the aforementioned peptide and inhibits the cleavage of XIP by caspase-1.

A further aspect of the present invention relates to a pharmaceutical composition comprising the aforementioned agent for inhibiting the degradation of XIP.

A further aspect of the present invention relates to an agent for preventing and/or treating a disease attributable to the degradation of hepatitis B virus x interacting protein (XIP), wherein the agent comprises the aforementioned agent for inhibiting the degradation of XIP.

A still further aspect of the present invention relates to an agent for inhibiting the replication and/or transcription of hepatitis B virus (HBV), wherein the agent comprises the aforementioned agent for inhibiting the degradation of XIP.

A further aspect of the present invention relates to an anti-hepatitis B virus (HBV) agent, wherein the agent comprises the aforementioned agent for inhibiting the degradation of XIP.

A further aspect of the present invention relates to an agent for preventing and/or treating hepatitis B, comprising the aforementioned agent for inhibiting the degradation of XIP.

A further aspect of the present invention relates to an agent for preventing and/or treating hepatic cancer, wherein the agent comprises the aforementioned agent for inhibiting the degradation of XIP.

A still further aspect of the present invention relates to a method for inhibiting the degradation of hepatitis B virus x interacting protein (XIP), wherein the method comprises a process for inhibiting caspase-1.

A further aspect of the present invention relates to a method for inhibiting the degradation of hepatitis B virus x interacting protein (XIP), wherein the method comprises a process for inhibiting the cleavage of XIP caused by caspase-1.

A further aspect of the present invention relates to a method for preventing and/or treating a disease attributable to the degradation of hepatitis B virus x interacting protein (XIP), wherein the method comprises using the aforementioned method for inhibiting the degradation of XIP.

A still further aspect of the present invention relates to a method for inhibiting the replication and/or transcription of hepatitis B virus (HBV), wherein the method comprises using the aforementioned method for inhibiting the degradation of XIP.

A further aspect of the present invention relates to a method for preventing and/or treating hepatitis B, wherein the method comprises using the aforementioned method for inhibiting the degradation of XIP.

A further aspect of the present invention relates to a method for preventing and/or treating hepatic cancer, wherein the method comprises using the aforementioned method for inhibiting the degradation of XIP.

A still further aspect of the present invention relates to a method for preventing and/or treating a disease attributable to the degradation of hepatitis B virus x interacting protein (XIP), wherein the method comprises using the aforementioned agent for inhibiting the degradation of XIP.

A further aspect of the present invention relates to a method for inhibiting the replication and/or transcription of hepatitis B virus (HBV), wherein the method comprises using the aforementioned agent for inhibiting the degradation of XIP.

A further aspect of the present invention relates to a method for preventing and/or treating hepatitis B, wherein the method comprises using the aforementioned agent for inhibiting the degradation of XIP.

A still further aspect of the present invention relates to a method for preventing and/or treating hepatic cancer, wherein the method comprises using the aforementioned agent for inhibiting the degradation of XIP.

A further aspect of the present invention relates to a method for identifying an inhibitory agent for the degradation of hepatitis B virus x interacting protein (XIP), wherein the method comprises contacting caspase-1 and/or XIP with a compound under conditions that allow the cleavage of XIP by caspase-1, introducing a system using a signal and/or a marker capable of detecting the degradation of XIP, detecting the presence or absence and/or change of the signal and/or the marker, and determining whether the compound inhibits the degradation of XIP.

A further aspect of the present invention relates to a method for identifying an inhibitory agent for the degradation of hepatitis B virus x interacting protein (XIP), wherein the method comprises contacting caspase-1 and/or XIP with a compound under conditions that allow the cleavage of XIP by caspase-1, detecting the presence or absence and/or change of the amount of XIP or the amount of XIP degradation product, and determining whether the compound inhibits the degradation of XIP.

A still further aspect of the present invention relates to a compound identified by the aforementioned identification method.

A further aspect of the present invention relates to a kit containing at least one member selected from the group consisting of caspase-1, a polynucleotide encoding caspase-1, and a vector containing a polynucleotide encoding caspase-1, and at least one member selected from the group consisting of hepatitis B virus x interacting protein (XIP), a polynucleotide encoding XIP, and a vector containing a polynucleotide encoding XIP.

### BRIEF DESCRIPTION OF THE DRAWINGS

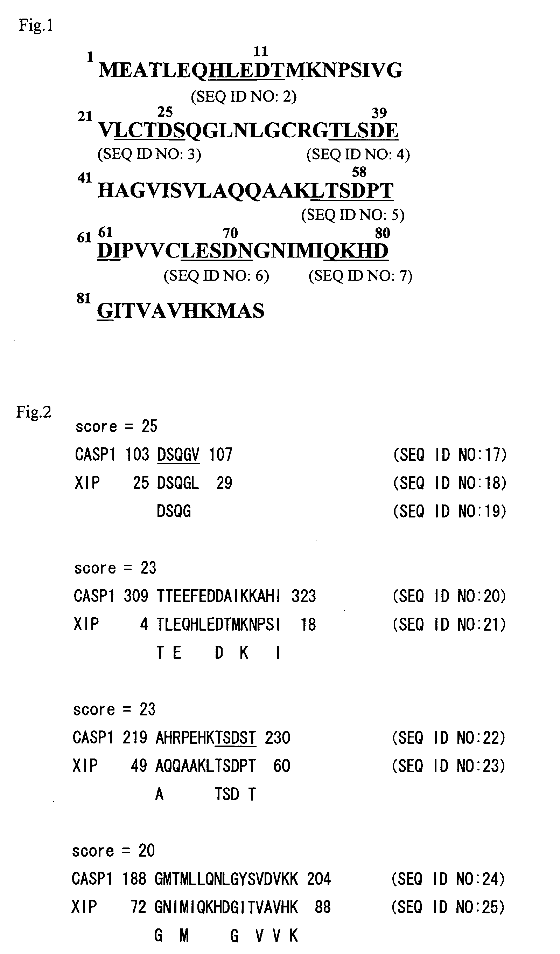

Fig. 1 shows the cleavage sites of XIP (SEQ ID NO: 1) recognized by caspase-1. The amino acid sequence is represented in the single-letter code, and the cleavage sites are indicated by the symbol of "/".

Fig. 2 shows the results of an *in-silico* prediction of the interaction of caspase-1 (CASP1) with XIP (SEQ ID NO: 1). A region that exhibited a high score as a result of local alignment between caspase-1 and XIP (SEQ ID NO: 1) is shown. The amino acid sequences are represented in the single-letter code. (Example 1)

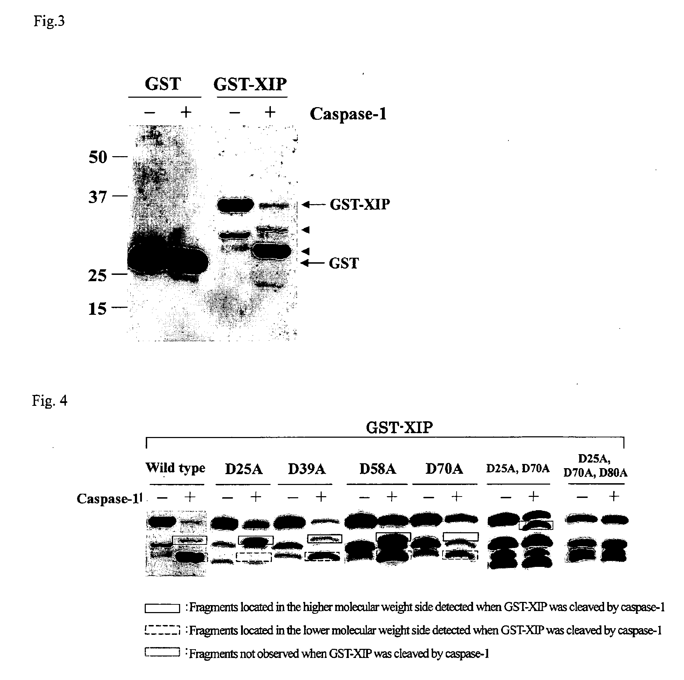

Fig. 3 shows that caspase-1 cleaved the fusion protein (GST-XIP) of glutathione S-transferase (GST) and XIP (SEQ ID NO: 1), but did not cleave GST. Symbols "+" and "-" shown in the figure respectively indicate the presence or absence of caspase-1. Each arrow indicates GST-XIP or GST as shown in the figure. Arrowheads indicate the fragments generated by the cleavage of GST-XIP by caspase-1. (Example 2)

Fig. 4 shows that a fragment located at the higher molecular weight side and/or a fragment located at the lower molecular weight side generated by the cleavage of the fusion protein (GST-XIP) of GST and XIP (SEQ ID NO: 1) by caspase-1 were found among fragments generated by the cleavage of the fusion protein of XIP variant and GST [GST-XIP (D25A), GST-XIP (D39A), GST-XIP (D58A), GST-XIP (D70A), and GST-XIP (D25A, D70A)] by caspase-1. Moreover, in the fragments of GST-XIP (D25A, D70A), a band other than the two bands indicating the aforementioned fragments and that indicates another fragment, was detected. This band was not detected in the fragments of GST-XIP (D25A, D70A and D80A). Symbols "+" and "-" shown in the figure respectively indicate the presence or absence of caspase-1. (Example 4)

### DETAILED DESCRIPTION OF THE INVENTION

The present invention claims the benefit of priority from Japanese Patent Application No. 2003-045711, which is incorporated herein by reference in its entirety.

Technical and scientific terms used herein have the meanings as normally understood by those skilled in the art, unless otherwise defined. Various methods that are well known to those skilled in the art are referenced herein. These reference materials, such as published materials disclosing known methods cited herein, are incorporated herein by reference in their entirety.

Embodiments of the present invention are explained in further detail below. However, the detailed description below is for the purpose of explanation only, and is not intended to limit the scope of the present invention.

In the present invention, the interaction of caspase-1 with XIP (SEQ ID NO: 1) was predicted *in-silico* according to the method described in International Publication No. WO O1/67299. Further, it was revealed by way of experiment, for the first time, that caspase-1 cleaves and degrades XIP (SEQ ID NO: 1).

The cleavage sequences recognized by caspase-1 in an amino acid sequence of XIP (SEQ ID NO: 1) were inferred to be HLED/T (SEQ ID NO: 2), LCTD/S (SEQ ID NO: 3), TLSD/E (SEQ ID NO: 4), LTSD/PTD/I (SEQ ID NO: 5), LESD/N (SEQ ID NO: 6) and QKHD/G (SEQ ID NO: 7) (see Fig.1). Herein, the amino acid sequences are represented by a single-letter code. The symbol "/" shown in the amino acid sequence indicates a cleavage site. Moreover, the term "the cleavage sequence recognized by caspase-1 (or the cleavage recognition sequence by caspase-1)" refers to a sequence that is recognized by caspase-1 and contains a cleavage site.

XIP (SEQ ID NO: 1) is known to bind to HBx and suppress the promoting effect thereof on HBV replication (Non-patent document 6). HBx is known to promote the transcription activity of various viral and cellular promoters and to activate an intracellular signal transduction pathway that plays a role in cell proliferation, in addition to the effect of promoting HBV replication. These facts suggest that XIP (SEQ ID NO: 1) is a protective factor suppressing the replication and/or transcription of a virus such as HBV and the aberrant proliferation of cells, by binding to HBx to suppress the effect thereof.

The present inventors have considered that caspase-1 cleaves XIP (SEQ ID NO: 1) to degrade it, resulting in the recovery of the suppressed HBx effect, which then causes the replication and/or transcription of a virus such as HBV and the aberrant proliferation of cells. For example, the present inventors have considered that the mechanism for the occurrence and progression of hepatitis B and the development of hepatic cancer therefrom includes a pathway in which caspase-1 cleaves XIP (SEQ ID NO: 1) to degrade it, resulting in eliminating the suppressive effect of XIP (SEQ ID NO: 1) on HBV replication.

Therefore, by inhibiting the cleavage of XIP (SEQ ID NO: 1) caused by caspase-1, it is possible to suppress the HBx action and thereby to suppress the replication and/or transcription of HBV and the aberrant proliferation of cells. Furthermore, it is possible to prevent and/or treat a disease attributable to the degradation of XIP (SEQ ID NO: 1). Particularly, for example, it is possible to inhibit the occurrence and progression of hepatitis B and the development of hepatic cancer therefrom.

One aspect of the present invention that was achieved based on these findings relates to an agent for inhibiting the degradation of XIP. The agent for inhibiting the degradation of XIP preferably has a feature of inhibiting the cleavage of XIP (SEQ ID NO: 1) caused by caspase-1.

Herein, the term "the degradation of XIP (SEQ ID NO: 1)" refers to a degradation where a peptide bond of XIP (SEQ ID NO: 1) is cleaved at a certain site in its amino acid sequence, resulting in fragmentation of XIP (SEQ ID NO: 1). Further, the term "an inhibitory agent" refers to a compound (exemplified by a peptide, an antibody and a low molecular weight compound described later that have a competitive inhibitory effect) that exerts an inhibitory effect on a certain function, or a composition containing the compound. Specifically, the term "an agent for inhibiting the degradation of XIP" refers to a compound that exerts an inhibitory effect on the degradation of XIP (SEQ ID NO: 1) or a composition containing the compound.

The agent for inhibiting degradation according to the present invention comprises a compound that inhibits the degradation of XIP (SEQ ID NO: 1), preferably a compound that inhibits the cleavage of XIP (SEQ ID NO: 1) by caspase-1. More preferably, it comprises a compound that inhibits specifically the cleavage of XIP (SEQ ID NO: 1) by caspase-1. The term "a compound that inhibits specifically the cleavage of XIP (SEQ ID NO: 1) by caspase-1" refers to a compound that strongly inhibits the cleavage of XIP (SEQ ID NO: 1) by caspase-1, but does not inhibit or only slightly inhibits the cleavage of other proteins by caspase-1.

Examples of such a compound include a compound that inhibits an enzyme activity of caspase-1 and a compound that inhibits the interaction of caspase-1 with XIP (SEQ ID NO: 1). Herein, the term "interaction of caspase-1 with XIP (SEQ ID NO: 1)" refers to a process wherein caspase-1 and XIP (SEQ ID NO: 1) exert an effect on each other in a certain manner, resulting in XIP (SEQ ID NO: 1) being cleaved and degraded. The term "a certain manner" described above includes binding, contacting, and being close, and may be any manner as long as it results in the interaction.

A compound that inhibits the interaction of caspase-1 with XIP (SEQ ID NO: 1) can be exemplified particularly by a peptide comprising the amino acid sequence of a region where the both proteins interact. A peptide derived from XIP (SEQ ID NO: 1) that is a substrate of caspase-1 is particularly useful because it competitively inhibits the interaction of both proteins and can inhibit the cleavage of XIP (SEQ ID NO: 1) by caspase-1. Such a peptide can be identified by firstly designing peptides based on the amino acid sequence of caspase-1 or XIP (SEQ ID NO: 1), synthesizing them by peptide synthesis methods well-known in the art, and selecting a peptide that inhibits the cleavage of XIP (SEQ ID NO: 1) by caspase-1. Selection can be carried out using a method for identifying a compound that can inhibit the degradation of XIP (SEQ ID NO: 1) as described later. Herein, the term "a peptide" refers to a substance comprising two or more amino acids mutually binding by a peptide bond or a modified peptide bond, such as a polypeptide or an oligopeptide.

Such a peptide is preferably exemplified by a peptide consisting of a cleavage sequence of XIP (SEQ ID NO: 1) recognized by caspase-1. Particularly, HLED/T (SEQ ID NO: 2), LCTD/S (SEQ ID NO: 3), TLSD/E (SEQ ID NO: 4), LTSD/PTD/I (SEQ ID NO: 5), LESD/N (SEQ ID NO: 6), and QKHD/G (SEQ ID NO: 7) can be exemplified.

Since it has been experimentally clarified that caspase-1 cleaves XIP (SEQ ID NO: 1) at the C-terminal side of at least the 25^{th}, the 70^{th}, and the 80^{th} aspartic acids (D), a peptide is more preferable which consists of a partial sequence containing any of these aspartic acids within the amino acid sequence of XIP (SEQ ID NO: 1). LCTD/S (SEQ ID NO: 3), LESD/N (SEQ ID NO: 6), and QKHD/G (SEQ ID NO: 7) can be exemplified respectively for the peptides containing the 25^{th}, the 70^{th}, and the 80^{th} aspartic acids (D) of XIP (SEQ ID NO: 1).

Furthermore, a peptide containing any one or more amino acid sequences selected from those set forth in SEQ ID NOS: 2-7 can be also used similarly as an effective ingredient of an agent for inhibiting the degradation of XIP. XIP (SEQ ID NO: 1) itself is not included within the range of a peptide that can be used for an agent for inhibiting the degradation of XIP. Moreover, a peptide having an amino acid sequence of the aforementioned peptide, in which a mutation such as a deletion, substitution, addition, or insertion of one or several amino acids was introduced, can be also used as an effective ingredient of the aforementioned inhibitory agent. The peptide containing any one or more amino acid sequences selected from those set forth in SEQ ID NOS: 2-7, or the peptide described above into which a mutation has been introduced is preferably a peptide that inhibits the cleavage of XIP (SEQ ID NO: 1) by caspase-1. The peptide into which a mutation has been introduced may be a naturally existing peptide or a peptide into which a mutation was artificially introduced. A means for introducing a mutation such as a deletion, substitution, addition, or insertion is known by itself. For example, the Ulmer technique (Non-patent document 7) can be used. When introducing such a mutation, mutual substitution among homologous amino acids (polar amino acids, non-polar amino acids, hydrophobic amino acids, hydrophilic amino acids, positively-charged amino acids, negatively-charged amino acids and aromatic amino acids and the like) may be readily conceived in view of avoiding a change in the fundamental properties (such as physical properties, function, or immunological activity) of the peptide. Furthermore, these usable peptides can be modified to the extent that no significant functional change is involved, such as modification of its constituent amino group or carboxyl group and the like by an amidation and the like.

The aforementioned peptide can be produced by common methods that are known in peptide chemistry. A method described in the References (Non-patent documents 8 and 9), for example, may be used, although the methods are not limited thereto and known methods can be broadly utilized. Particularly, a peptide synthesis method using an ordinary liquid phase method and solid phase method, such as, for example, the Fmoc method, can be exemplified. Moreover, an amino acid synthesizer that is commercially available can be used for producing the peptide. Alternatively, a gene engineering technology can be also used for producing the peptide. For example, a gene encoding a peptide of interest can be used to prepare a recombinant DNA (expression vector) that can express the gene in a host cell, followed by transfection of the recombinant DNA into a suitable host cell such as E. coli to obtain a transformant. Culturing the transformant and collecting the peptide of interest from the obtained culture product achieves the production of the peptide.

A compound for inhibiting the degradation of XIP (SEQ ID NO: 1) can be exemplified by an antibody that recognizes caspase-1 or XIP (SEQ ID NO: 1) and inhibits the cleavage of XIP (SEQ ID NO: 1) by caspase-1, in addition to the aforementioned compound. An antibody can be produced by known methods for preparing an antibody using caspase-1 or XIP (SEQ ID NO: 1) itself, a partial peptide derived from these, or a peptide comprising the amino acid sequence of an interaction site of these, as an antigen.

A compound for inhibiting the degradation of XIP (SEQ ID NO: 1) can be further exemplified by a compound that is obtained by an identification method described later, preferably such a compound with a low molecular weight.

One aspect of the present invention relates to a method for identifying a compound that inhibits the degradation of XIP (SEQ ID NO: 1). The method for identifying a compound that inhibits the degradation of XIP (SEQ ID NO: 1) can be constructed utilizing a known pharmaceutical screening system by using caspase-1 or XIP (SEQ ID NO: 1), genes encoding thereof, a vector into which the gene has been inserted, a transformant into which the vector has been introduced, or a cell in which the gene is expressed, alone or in combination.

Caspase-1 or XIP (SEQ ID NO: 1) for use in the method for identifying a compound can be prepared by a chemical synthesis method or a gene engineering technology that is generally known. Specifically, these can be the chemical synthesis products or the products of cell-free synthesis systems, or can be cells in which these are expressed or artificially expressed, or can be the products prepared from the cells or from any biological samples, or can be the products further purified by the aforementioned products. Moreover, these proteins can be labeled by ligating a labeling substance thereto at the N-terminus or the C-terminus as long as it has no influence upon the interaction of caspase-1 with XIP (SEQ ID NO: 1) and the functions of these proteins, such as the enzyme activity of caspase-1 on protein degradation and the property of XIP (SEQ ID NO: 1) as an enzyme substrate. Examples of the labeling substance include other proteins or polypeptides such as GST, β-galactosidase, an immunoglobulin Fc fragment (such as IgG), a tag peptide (such as His-tag, Myc-tag, HA-tag, FLAG-tag, or Xpress-tag), biotin, and a radioactive isotope. These labeling substances can be ligated directly or indirectly via a linker peptide and the like, by means of, for example, genetic engineering techniques. Caspase-1 or XIP (SEQ ID NO: 1) can be easily purified or detected by determining these labeling substances themselves or their functions. Moreover, these labeling substances can be utilized, for example, for the purpose of detecting the interaction of caspase-1 or XIP (SEQ ID NO: 1).

The aforementioned identification method can be carried out by selecting conditions that allow for the cleavage of XIP (SEQ ID NO: 1) by caspase-1, contacting a compound to be tested (a test compound) with caspase-1 and/or XIP (SEQ ID NO: 1) under the conditions, employing a system that uses a signal and/or a marker capable of detecting degradation of XIP (SEQ ID NO: 1), and then detecting the presence, the absence and/or the change of the signal and/or the marker. The conditions that allow for the cleavage of XIP (SEQ ID NO: 1) by caspase-1 may be an *in vitro* or *in vivo* condition. For example, a cell in which capase-1 is co-expressed with XIP (SEQ ID NO: 1) may be also used. Contact of the test compound with caspase-1 and/or XIP (SEQ ID NO: 1) may be conducted prior to a degradation reaction of XIP (SEQ ID NO: 1) by caspase-1, or may be conducted by allowing the test compound to coexist in the degradation reaction. Herein, the signal refers to a substance that can be detected directly based on its physical properties or chemical properties, and the marker refers to a substance that can be detected indirectly by using the physical properties or biological properties as an indicator. Examples of the signal include luciferase, a green fluorescent protein, and a radioactive isotope. Examples of the marker include a reporter gene such as a chloramphenicol acetyl transferase gene. A known signal such as epitope tags for detection, for example, 6 x His-tag and the like can be used. Methods for detecting these signals or markers are well known to those skilled in the art. As a convenient method, degradation of XIP (SEQ ID NO: 1) can be detected by determining the presence, the absence and/or the change of the amount of XIP (SEQ ID NO: 1) or the amount of a degradation product of XIP (SEQ ID NO: 1). Determination of the amount of the XIP (SEQ ID NO: 1) or the amount of a degradation product of XIP (SEQ ID NO: 1) can be carried out using a known method for detecting a protein or peptide, such as, for example, western blotting.

Examples of a test compound include a compound derived from a chemical library or a natural product, as well as a compound obtained by drug design based on the primary structure or tertiary structure of caspase-1 and XIP (SEQ ID NO: 1). Alternatively, a compound obtained by drug design based on the structure of a peptide of a recognized cleavage site of XIP (SEQ ID NO: 1) recognized by caspase-1 or the like is also suitable as a test compound.

The aforementioned peptides and the compounds obtained by the aforementioned identification method are included in the scope of the present invention. The aforementioned peptides and/or the aforementioned compounds can be used for producing an agent for inhibiting the degradation of XIP according to the present invention, when used alone or in combination of two or more. For example, an agent for inhibiting the degradation of XIP that is produced by using the peptides in a combination of two or more can inhibit the cleavage of XIP (SEQ ID NO: 1) by caspase-1 at several cleavage sites, and hence may bring a significant effect in the inhibition of the degradation of XIP (SEQ ID NO: 1). In addition, these peptides and compounds may be used as a reagent. These are useful, for example, for a study at a molecular level of the involvement of XIP (SEQ ID NO: 1) and HBx in the mechanism for the occurrence and progression of hepatitis B and the development of hepatic cancer therefrom.

An agent for inhibiting the degradation of XIP that comprises the aforementioned peptide and/or the aforementioned compound can be selected in consideration of a balance between the biological usefulness and toxicity to prepare a pharmaceutical composition. Such a pharmaceutical composition is also included in the scope of the present invention.

One aspect of the present invention relates to an agent for preventing and/or treating a disease attributable to the degradation of XIP (SEQ ID NO: 1), wherein the agent inhibits the cleavage of XIP (SEQ ID NO: 1) by caspase-1. It is possible to achieve the inhibition of the cleavage of XIP (SEQ ID NO: 1) caused by caspase-1, for example, by means of inhibiting the effect of caspase-1 or inhibiting the interaction of caspase-1 with XIP (SEQ ID NO: 1). The inhibition of the effect of caspase-1 can be carried out, for example, by means of inhibiting the enzyme activity of caspase-1. The inhibition of enzyme activity of caspase-1 can be carried out by identifying a compound capable of inhibiting the activity using an identification method according to the present invention and then using the compound therefor. Moreover, it can be also carried out by utilizing a known inhibitor of the enzyme activity of caspase-1. The inhibition of the interaction of caspase-1 with XIP (SEQ ID NO: 1) can be carried out, for example, by utilizing a peptide according to the present invention and/or a compound obtained by the aforementioned identification method. Specifically, the aforementioned preventing and/or treating agent can be exemplified by a preventing and/or treating agent comprising at least one of the aforementioned compounds and the aforementioned peptides.

Since XIP (SEQ ID NO: 1) is involved in the suppression of HBx-induced HBV replication and transcription, hepatitis B is exemplified as a disease attributable to the degradation of XIP (SEQ ID NO: 1). It is possible to suppress the HBx-induced HBV replication and transcription by inhibiting the cleavage of XIP (SEQ ID NO: 1) by caspase-1, which enables it to prevent and/or treat hepatitis B. Moreover, prevention and/or treatment of hepatic cancer developed from hepatitis B can be carried out by inhibiting the cleavage of XIP (SEQ ID NO: 1) by caspase-1. In view of these facts, an agent for inhibiting the replication and/or transcription of HBV and an anti-hepatitis B virus agent are also included in the scope of the present invention, wherein the agents inhibit the cleavage of XIP (SEQ ID NO: 1) by caspase-1. Moreover, an agent for preventing and/or treating hepatitis B and an agent for preventing and/or treating hepatic cancer are also included in the scope of the present invention, wherein the agents inhibit the cleavage of XIP (SEQ ID NO: 1) by caspase-1. Particularly, an inhibitory agent and an agent for preventing and/or treating a disease comprising at least one of the aforementioned compounds and the aforementioned peptides can be exemplified.

Furthermore, the agent for inhibiting the degradation of XIP according to the present invention can be utilized as an effective ingredient of the agent for preventing and/or treating a disease attributable to the degradation of XIP (SEQ ID NO: 1). The aforementioned agent for inhibiting the degradation of XIP can be used for an effective ingredient of the agent for inhibiting the replication and/or transcription of HBV and an anti-hepatitis B virus agent. Moreover, the aforementioned agent for inhibiting the degradation of XIP is suitable for an effective ingredient of an agent for preventing and/or treating occurrence and progression of hepatitis B and hepatic cancer.

A suitable carrier (pharmaceutical carrier) that is pharmaceutically acceptable may be preferably contained in the agent for inhibiting the degradation of XIP, the agent for inhibiting the replication and/or transcription of HBV, the anti-hepatitis B virus agent, and the agent for preventing and/or treating a disease attributable to the degradation of XIP (SEQ ID NO: 1), such as hepatitis B and hepatic cancer, according to the present invention. Examples of the pharmaceutical carrier include diluent or excipient, such as a filler, extender, binder, wetting agent, disintegrator, surfactant, and lubricant that are generally used in accordance with the dosage form of the formulation. The carrier is exemplified by water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose, but it is not limited thereto. One or a combination of two or more kinds of these may be suitably selected and used in accordance with the dosage form. As desired, various components that can be used in conventional protein preparation, such as a stabilizer, bacteriocide, buffer agent, isotonizing agent, chelating agent, or pH adjuster, can be suitably contained in the formulation.

The amount of the effective ingredient contained in the agent for inhibiting the degradation of XIP, the agent for inhibiting the replication and/or transcription of HBV, the anti-hepatitis B virus agent, and the agent for preventing and/or treating a disease attributable to the degradation of XIP (SEQ ID NO: 1) such as hepatitis B and hepatic cancer according to the present invention can be appropriately selected from a broad range. In general, a suitable amount may fall within a range of approximately 0.00001 to 70 wt%, preferably approximately 0.0001 to 5 wt%.

Suitable dosage ranges are not particularly limited, and can be determined according to the following: effectiveness of the ingredients contained therein; the administration form; the route of administration; the type of disease; the characteristics of the subject (e.g., body weight, age, symptomatic conditions and whether being taking other medicament) and the judgment of the physician in charge. In general, a suitable dosage may fall, for example, within a range of approximately 0.01 µg to 100 mg per 1 kg of the body weight of the subject, and preferably within a range of approximately 0.1 µg to 1 mg per 1 kg. However, a dosage may be altered using conventional experiments for optimization of a dosage that are well known in the art. The aforementioned dosage can be divided for administration once to several times a day. Alternatively, periodic administration once every few days or few weeks can be employed. Moreover, other compounds or medicaments necessary for the treatment may be used together.

In terms of a route of administration, both systemic administration and local administration can be selected. In this case, an appropriate route of administration is selected in accordance with diseases, symptoms or other factors. As examples, parenteral administration including normal intravenous injection, intraarterial administration, subcutaneous administration, intracutaneous administration and intramuscular administration can be employed. Oral administration can be also employed. Further, transmucosal administration or dermal administration can be employed. When using it for a neoplastic disease, direct administration to the neoplasm by an injection or the like is preferable.

In terms of a dosage form, various administration forms can be selected in accordance with a treatment purpose, and typical examples thereof include an administration form of solid formulation such as a tablet, a pill, powder, powdered drug, fine granule, granule or a capsule, as well as an administration form of liquid formulation such as an aqueous formulation, ethanol formulation, suspension, fat emulsion, liposome formulation, clathrate such as cyclodextrin, a syrup and an elixir. These can be further classified according to the administration route into oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

Powders, pills, capsules, and tablets can be prepared using an excipient such as lactose, glucose, sucrose, or mannitol; a disintegrant agent such as starch or sodium alginate; a lubricant such as magnesium stearate or talc; a binder such as polyvinyl alcohol, hydroxypropyl cellulose, or gelatin; a surfactant such as fatty acid ester; a plasticizer such as glycerin, and the like. For preparation of a tablet or a capsule, a pharmaceutical carrier in a solid state is used.

A suspension can be prepared using water; sugars such as sucrose, sorbitol, or fructose; glycols such as polyethylene glycol; and oils.

Injectable solutions can be prepared using a carrier comprising a salt solution, a glucose solution or a mixture of salt water and a glucose solution.

Inclusion into a liposome formulation can be conducted in the following manner: by dissolving the aforementioned effective ingredient in a solvent (e.g., ethanol) to make a solution, adding a solution of phospholipids dissolved in an organic solvent (e.g., chloroform), removing the solvent by evaporation and adding a phosphate buffer thereto, agitating the solution and then subjecting it to sonication followed by centrifugation to obtain a supematant, and finally, filtrating the supernatant to recover liposomes.

A fat emulsion can be prepared in the following manner: by mixing the aforementioned effective ingredient, an oil ingredient (e.g., vegetable oil such as soybean oil, sesame oil, olive oil, or MCT), an emulsifier (such as a phospholipid), and the like; heating the mixture to make a solution; adding water of a required quantity; and then emulsifying or homogenizing by use of an emulsifier (a homogenizer, e.g., a high pressure jet type, an ultrasonic type, or the like). The fat emulsion may be also lyophilized. For conducting lipid-emulsification, an auxiliary emulsifier may be added, and examples thereof include glycerin or saccharides (e.g., glucose, sorbitol, fructose, etc.).

Inclusion into a cyclodextrin formulation may be carried out in the following manner: by dissolving the aforementioned effective ingredient in a solvent (e.g., ethanol); adding a solution of cyclodextrin dissolved in water under heating thereto; chilling the solution and filtering the precipitates; and drying under sterilization. At this time, the cyclodextrin to be used may be appropriately selected from among those having different void sizes (α, β, or γ type) in accordance with the bulkiness of the substance of interest.

One aspect of the present invention also relates to a method for inhibiting the degradation of XIP (SEQ ID NO: 1). The method for inhibiting the degradation of XIP (SEQ ID NO: 1) according to the present invention can be carried out, for example, by inhibiting the effect of caspase-1. Alternatively, the method for inhibiting the degradation of XIP (SEQ ID NO: 1) can be achieved by inhibiting the interaction of caspase-1 with XIP (SEQ ID NO: 1). The inhibition of the effect of caspase-1 and the inhibition of the interaction of caspase-1 with XIP (SEQ ID NO: 1) can be carried out as described above.

By using the method for inhibiting the degradation of XIP (SEQ ID NO: 1) according to the present invention, a method for preventing and/or treating a disease attributable to the degradation of XIP (SEQ ID NO: 1) can be carried out. Since XIP (SEQ ID NO: 1) suppresses the HBx-induced HBV replication and transcription, a method for inhibiting the replication and/or transcription of HBV can be achieved by using the aforementioned method for inhibiting the degradation of XIP (SEQ ID NO: 1). Furthermore, a method for preventing and/or treating hepatitis B and hepatic cancer attributable to hepatitis B can be carried out by using the aforementioned method for inhibiting the degradation of XIP (SEQ ID NO: 1).

The inhibition method and the prevention and/or treatment method can be also carried out using the aforementioned agent for inhibiting the degradation of XIP. For example, the aforementioned prevention and/or treatment method can be achieved by administering the aforementioned agent for inhibiting the degradation of XIP to a patient suffering from a disease attributable to the degradation of XIP (SEQ ID NO: 1).

The aforementioned inhibition method and the prevention and/or treatment method for a disease are also included in the scope of the present invention.

One aspect of the present invention further relates to a reagent kit. The reagent kit of the present invention contains at least one member selected from the group consisting of caspase-1, a polynucleotide encoding caspase-1, and a vector containing the polynucleotide encoding caspase-1; and at least one member selected from the group consisting of XIP (SEQ ID NO: 1), a polynucleotide encoding XIP (SEQ ID NO: 1) and a vector containing the polynucleotide encoding XIP (SEQ ID NO: 1). The aforementioned kit may be used, for example, for the identification method according to the present invention.

Caspase-1 and XIP (SEQ ID NO: 1) can be obtained by a chemical synthesis method or a gene engineering technology that is generally known as described above.

The polynucleotide that encodes caspase-1 or XIP (SEQ ID NO: 1) can be prepared from a human cDNA library by using a known gene engineering method. The vector containing the polynucleotide encoding caspase-1 or XIP (SEQ ID NO: 1) is obtained by introducing the aforementioned polynucleotide into an appropriate expression DNA vector such as a bacterial plasmid derived vector by a known gene engineering method.

The aforementioned kit may include a desired substance such as a signal and/or a marker for detecting the degradation of XIP (SEQ ID NO: 1), buffer solutions, and salt. Furthermore, a substance such as a stabilizer and/or an antiseptic agent may be contained. At the time of preparation, methods for preparation in accordance with the respective substances to be used may be introduced.

### EXAMPLES

Hereinafter, the present invention may be explained more particularly with examples; however, the present invention is not limited to the following examples.

### Example 1

### (In-silico search of interaction of caspase-1 with XIP (SEQ ID NO: 1))

The prediction of proteins that interact with caspase-1 was conducted *in-silico* according to the prediction method described in International Publication No. WO 01/67299 gazette. First, the amino acid sequence of caspase-1 was decomposed into peptides having a pre-determined length in order to search in a database for proteins having the amino acid sequence of each of the peptides, or having homologous amino acid sequences to these amino acid sequences. Subsequently, local alignment was conducted between the proteins obtained and caspase-1 to identify proteins having a high local alignment score that might be capable of interacting with caspase-1.

As a result of analysis, it was found that the peptides TSDPT and DSQGL (SEQ ID NO. 18), which have homology respectively to the peptides TSDST and DSQGV (SEQ ID. NO. 17) that are the partial sequence of caspase-1 consisting of 5 amino acid residues, are present in the amino acid sequence of XIP (SEQ ID NO: 1) (Fig. 2). Herein, amino acid sequences are represented in the single-letter code. Further, the local alignment between caspase-1 and XIP (SEQ ID NO: 1) resulted in findings of four regions with the scores of 20 or more (Fig. 2). This result lead us to a prediction that XIP (SEQ ID NO: 1) is a protein that may interact with caspase-1.

### Example 2

### (Analysis of the cleavage of XIP by caspase-1)

### <Materials>

cDNA of human XIP (SEQ ID NO: 1) was obtained from a human brain cDNA library by reverse transcription polymerase chain reaction (RT-PCR). The cDNA of human XIP (SEQ ID NO: 1) was inserted into the expression vector for E. coli, pGEX-4T (Amersham, Co., Ltd.) to construct an expression plasmid of XIP (SEQ ID NO: 1), pGEX-4T-XIP. This expression plasmid was used to prepare a fusion protein of XIP (SEQ ID NO: 1) and GST-tag in which the GST-tag was ligated at the N-terminus of XIP (hereinafter, referred to as GST-XIP). An amino acid sequence encoded by the cloned XIP cDNA was identical to the sequence of Accession Number NP_006393 in the NCBI protein data base.

### <Method>

The preparation and purification of GST-XIP was conducted as follows. First, an E. coli containing pGEX-4T-XIP was incubated until it was in the logarithmic growth phase (OD₆₀₀=0.6-0.8) at 25°C. Isopropyl-1-thio-beta-D-galactopyranoside (IPTG) was then added thereto at a final concentration of 0.1 mM, followed by incubating for four hours at 25°C to induce expression. The bacteria was collected and washed by phosphate buffered saline (PBS), and then suspended in the lysis buffer (50mM Tris-HCl, pH 7.5 / 1mM dithiothreitol (DTT) / 1 mM ethylenediaminetetraacetate/ 1 mM phenylmethylsulfonyl fluoride), followed by addition of lysozyme at a final concentration of 0.5 mg/ml, and left to stand on ice for 30 minutes. After adding Nonidet P-40 at a final concentration of 1%, the resultant was subjected to sonication, followed by centrifugation to collect the supernatant. GST-XIP was purified from this supernatant using Glutathione-Sepharose 4B. The purified GST-XIP was dialyzed with the dialysis solution (50 mM HEPES, pH 7.5 / 0.1 % CHAPS / 10mM DTT), and stored at -80°C until the time of use.

A caspase-1 protease assay was conducted as follows: GST-XIP was added to the reaction solution containing 50 mM HEPES, pH 7.5, 0.1% CHAPS and 10mM DTT in the presence or absence of caspase-1 (Calbiochem, Co., Ltd.) (a total amount of the reaction solution: 10µl) for reaction at 37°C for 2 hours. For the negative control, the same reaction as described above was conducted by using GST instead of GST-XIP. After the reaction, 10µl of 2 x sodium dodecyl sulfate (SDS) sample buffer was added, followed by heating for 2 minutes at 100°C and leaving to stand for 2 minutes on ice. 10µl of the aliquot was subjected to SDS-PAGE (gel concentration 10%), and then western blotting using the anti-GST antibody (Amersham, Co., Ltd.) was conducted to detect GST and GST-XIP.

### <Results>

As a result of the reaction of caspase-1 with GST-XIP, the reduction of the band that indicates GST-XIP and the increase of fragments accompanied therewith were observed (Fig. 3). On the other hand, GST was not cleaved by caspase-1. Hence, it was revealed that caspase-1 cleaves XIP (SEQ ID NO: 1) and degrades it.

### Example 3

The amino acid sequence of a region that is recognized and cleaved by caspase-1 (recognized cleavage sequence) in XIP (SEQ ID NO: 1) was studied. The known cleavage recognition sequences in known substrates of caspase-1 are as follows: YVHD/ A (SEQ ID NO: 8) in prointerleukin-1β; LESD / N (SEQ ID NO: 9) in prointerleukin-18; ALDD / L (SEQ ID NO: 10), LSSD / F (SEQ ID NO: 11) and ALAD / S (SEQ ID NO: 12) in calpastatin; YVPD / S (SEQ ID NO: 13) in PITSLRE kinase; IETD / S (SEQ ID NO: 14) in caspase-3; and LVCD / N (SEQ ID NO: 15) and ELPD / G (SEQ ID NO: 16) in actin. Herein, the symbol "/" represents the cleavage site. Considering the homology with these sequences, it was presumed that the cleavage sequences of XIP (SEQ ID NO: 1) recognized by caspase-1 were HLED /T (SEQ ID NO: 2), LCTD / S (SEQ ID NO: 3), TLSD/E (SEQ ID NO: 4), LTSD / PTD / I (SEQ ID NO: 5), LESD/N (SEQ ID NO: 6) and QKHD / G (SEQ ID NO: 7) (Fig. 1). This result supports the conclusion that XIP (SEQ ID NO: 1) is degraded by caspase-1.

### Example 4

### (Identification of cleavage sites of XIP by caspase-1)

### <Materials>

Various GST-XIP variant expression plasmids were prepared for expressing GST-XIP variants that are the XIP variants having one or several substitution(s) of aspartic acid (D) for alanine (A) in the amino acid sequence of XIP (SEQ ID NO: 1) and being tagged with GST at the N-terminal. Using the XIP expression plasmid pGEX-4T-XIP prepared in Example 2 as a template, these variant expression plasmids was prepared using Quik Change Site-Directed Mutagenesis Kit (STRATAGENE, Co., Ltd.).

The aforementioned GST-XIP variant expression plasmids were used to prepare GST-XIP variants: a GST-XIP variant having a substitution of aspartic acid (D) for alanine (A) at the 25^{th}, the 39^{th}, the 58^{th} or the 70^{th} residue from the first methionine in the amino acid sequence of XIP (SEQ ID NO: 1); a GST-XIP variant having two substitutions of aspartic acid (D) for alanine (A) at the 25^{th} and the 70^{th} residues; and a GST-XIP variant having three substitutions of aspartic acid (D) for alanine (A) at the 25^{th}, the 70^{th} and the 80^{th} residues.

### <Method>

Each GST-XIP variant was prepared and purified using the aforementioned GST-XIP variant expression plasmids by the same method as described in Example 2. Hereinafter, each GST-XIP variant having a substitution of aspartic acid (D) for alanine (A) at the 25^{th}, the 39^{th}, the 58^{th} or the 70^{th} residue of XIP is referred to as GST-XIP (D25A), GST-XIP (D39A), GST-XIP (D58A) or GST-XIP (D70A), respectively. The GST-XIP variant having two substitutions of aspartic acid (D) for alanine (A) at the 25^{th} and the 70^{th} residues is referred to as GST-XIP (D25A, D70A). The GST-XIP variant having three substitutions of aspartic acid (D) for alanine (A) at the 25^{th}, the 70^{th} and the 80^{th} residues is referred to as GST-XIP (D25A, D70A, D80A).

A caspase-1 protease assay was conducted using GST-XIP and the aforementioned GST-XIP variants by the same method as described in Example 2.

### <Results>

The results are shown in Fig. 4. As a result of the caspase-1 protease assay, it was observed that GST-XIP, a wild type XIP, gave two bands, indicating the generation of two fragments. The fragment located at the higher molecular weight side of the fragments shown by these two bands was detected when using GST-XIP (D25A), GST-XIP (D39A), and GST-XIP (D58A), but not detected when using GST-XIP (D70A). On the other hand, the fragment located at the lower molecular weight side was detected when using GST-XIP (D39A), GST-XIP (D58A), GST-XIP (D70A), but not detected when using GST-XIP (D25A).

When using GST-XIP (D25A, D70A) as a substrate, a new fragment other than the aforementioned fragments was detected. This fragment was not detected when using GST-XIP (D25A, D70A, D80A) as a substrate.

The aforementioned results demonstrated that caspase-1 cleaves at the C-terminal side of at least the 25^{th}, the 70^{th}, and the 80^{th} aspartic acids (D) of XIP (SEQ ID NO: 1).

### INDUSTRIAL APPLICABILITY

In the present invention, the protein XIP (SEQ ID NO: 1), which interacts with caspase-1, was found. Then, it was revealed for the first time that caspase-1 cleaves and degrades XIP (SEQ ID NO: 1) as a result of the interaction, and further, the cleavage recognition sites have been identified. XIP (SEQ ID NO: 1) inhibits the effect of protein HBx derived from the HBV gene. HBx has a promoting effect on the replication of HBV and an activating effect on the transcription of the same, thereby, it is considered to be involved in the occurrence and progression of hepatitis B and the development of hepatic cancer therefrom. Considering these findings, the agent for inhibiting the degradation of XIP and/or the method for inhibiting the degradation of XIP that is provided in the present invention are extremely useful for inhibition of the replication and/or transcription of HBV, prevention and/or treatment of hepatitis B, and prevention and/or treatment of hepatic cancer attributable to HBV.

### FREE TEXT IN SEQUENCE LISTING

SEQ ID NO: 2: Partial sequence of hepatitis B virus x interacting protein (SEQ ID: NO.1), which is recognized by caspase-1.
SEQ ID NO: 3: Partial sequence of hepatitis B virus x interacting protein (SEQ ID NO: 1), which is recognized by caspase-1.
SEQ ID NO: 4: Partial sequence of hepatitis B virus x interacting protein (SEQ ID NO: 1), which is recognized by caspase-1.
SEQ ID NO: 5: Partial sequence of hepatitis B virus x interacting protein (SEQ ID NO: 1), which is recognized by caspase-1.
SEQ ID NO: 6: Partial sequence of hepatitis B virus x interacting protein (SEQ ID NO: 1), which is recognized by caspase-1.
SEQ ID NO: 7: Partial sequence of hepatitis B virus x interacting protein (SEQ ID NO: 1), which is recognized by caspase-1.
SEQ ID NO: 8: Partial sequence of prointerleukin-1 β, which is recognized by caspase-1.
SEQ ID NO: 9: Partial sequence of prointerleukin-18, which is recognized by caspase-1.
SEQ ID NO: 10: Partial sequence of calpastatin, which is recognized by caspase-1.
SEQ ID NO: 11: Partial sequence of calpastatin, which is recognized by caspase-1.
SEQ ID NO: 12: Partial sequence of calpastatin, which is recognized by caspase-1.
SEQ ID NO: 13: Partial sequence of PITSLRE kinase, which is recognized by caspase-1.
SEQ ID NO: 14: Partial sequence of caspase-3, which is recognized by caspase-1.
SEQ ID NO: 15: Partial sequence of actin, which is recognized by caspase-1.
SEQ ID NO: 16: Partial sequence of actin, which is recognized by caspase-1.
SEQ ID NO: 17: Partial sequence of caspase-1 showing a high score in the local alignment between caspase-1 and hepatitis B virus x interacting protein.
SEQ ID NO: 18: Partial sequence of hepatitis B virus x interacting protein showing a high score in the local alignment between caspase-1 and hepatitis B virus x interacting protein.
SEQ ID NO: 19: Partial sequence that is identical in the sequences of caspase-1 and hepatitis B virus x interacting protein.
SEQ ID NO: 20: Partial sequence of caspase-1 showing a high score in the local alignment between caspase-1 and hepatitis B virus x interacting protein.
SEQ ID NO: 21: Partial sequence of hepatitis B virus x interacting protein showing a high score in the local alignment between caspase-1 and hepatitis B virus x interacting protein.
SEQ ID NO: 22: Partial sequence of caspase-1 showing a high score in the local alignment between caspase-1 and hepatitis B virus x interacting protein.
SEQ ID NO: 23: Partial sequence of hepatitis B virus x interacting protein showing a high score in the local alignment between caspase-1 and hepatitis B virus x interacting protein.
SEQ ID NO: 24: Partial sequence of caspase-1 showing a high score in the local alignment between caspase-1 and hepatitis B virus x interacting protein.
SEQ ID NO: 25: Partial sequence of hepatitis B virus x interacting protein showing a high score in the local alignment between caspase-1 and hepatitis B virus x interacting protein.

## Claims

1. An agent for preventing and/or treating a disease attributable to the degradation of hepatitis B virus x interacting protein (XIP), wherein the agent inhibits the cleavage of hepatitis B virus x interacting protein (XIP) by caspase-1.

2. An agent for inhibiting the replication and/or transcription of hepatitis B virus (HBV), wherein the agent inhibits the cleavage of hepatitis B virus x interacting protein (XIP) by caspase-1.

3. An anti-hepatitis B virus (HBV) agent, wherein the agent inhibits the cleavage of hepatitis B virus x interacting protein (XIP) by caspase-1.

4. An agent for preventing and/or treating hepatitis B, wherein the agent inhibits the cleavage of hepatitis B virus x interacting protein (XIP) by caspase-1.

5. An agent for preventing and/or treating hepatic cancer, wherein the agent inhibits the cleavage of hepatitis B virus x interacting protein (XIP) by caspase-1.

6. A peptide consisting of an amino acid sequence set forth in SEQ ID NO: 2, 3, 4, 5, 6, or 7 in the sequence listing.

7. A peptide comprising an amino acid sequence set forth in SEQ ID NO: 2, 3, 4, 5, 6, or 7 in the sequence listing.

8. An agent for inhibiting the degradation of hepatitis B virus x interacting protein (XIP), wherein the agent comprises the peptide according to claim 6 or 7.

9. An agent for inhibiting the degradation of the hepatitis B virus x interacting protein (XIP), wherein the agent comprises the peptide according to claim 6 or 7 and inhibits the cleavage of XIP by caspase-1.

10. A pharmaceutical composition comprising the inhibitory agent according to claim 8 or 9.

11. An agent for preventing and/or treating a disease attributable to the degradation of hepatitis B virus x interacting protein (XIP), wherein the agent comprises the inhibitory agent according to claim 8 or 9.

12. An agent for inhibiting the replication and/or transcription of hepatitis B virus (HBV), wherein the agent comprises the inhibitory agent according to claim 8 or 9.

13. An anti-hepatitis B virus (HBV) agent, wherein the agent comprises the inhibitory agent according to claim 8 or 9.

14. An agent for preventing and/or treating hepatitis B, wherein the agent comprises the inhibitory agent according to claim 8 or 9.

15. An agent for preventing and/or treating hepatic cancer, wherein the agent comprises the inhibitory agent according to claim 8 or 9.

16. A method for inhibiting the degradation of hepatitis B virus x interacting protein (XIP), wherein the method comprises inhibiting caspase-1.

17. A method for inhibiting the degradation of hepatitis B virus x interacting protein (XIP), wherein the method comprises inhibiting the cleavage of XIP caused by caspase-1.

18. A method for preventing and/or treating a disease attributable to the degradation of hepatitis B virus x interacting protein (XIP), wherein the method comprises using the inhibition method according to claim 16 or 17.

19. A method for inhibiting the replication and/or transcription of hepatitis B virus (HBV), wherein the method comprises using the inhibition method according to claim 16 or 17.

20. A method for preventing and/or treating hepatitis B, wherein the method comprises using the inhibition method according to claim 16 or 17.

21. A method for preventing and/or treating hepatic cancer, wherein the method comprises using the inhibition method according to claim 16 or 17.

22. A method for preventing and/or treating a disease attributable to the degradation of hepatitis B virus x interacting protein (XIP), wherein the method comprises administering to a subject the inhibitory agent according to claim 8 or 9.

23. A method for inhibiting the replication and/or transcription of hepatitis B virus (HBV), wherein the method comprises administering to a subject the inhibitory agent according to claim 8 or 9.

24. A method for preventing and/or treating hepatitis B, wherein the method comprises administering to a subject the inhibitory agent according to claim 8 or 9.

25. A method for preventing and/or treating hepatic cancer, wherein the method comprises administering to a subject the inhibitory agent according to claim 8 or 9.

26. A method for identifying an inhibitory agent for the degradation of hepatitis B virus x interacting protein (XIP), wherein the method comprises contacting caspase-1 and/or XIP with a compound under conditions that allow the cleavage of XIP by caspase-1; introducing a system using a signal and/or a marker capable of detecting the degradation of XIP; detecting the presence or absence and/or change of the signal and/or the marker; and determining whether the compound inhibits the degradation of XIP.

27. A method for identifying an inhibitory agent for the degradation of hepatitis B virus x interacting protein (XIP), wherein the method comprises contacting caspase-1 and/or XIP with a compound under conditions that allow the cleavage of XIP by caspase-1; detecting the presence or absence and/or change of the amount of XIP or the amount of XIP degradation product; and determining whether the compound inhibits the degradation of XIP.

28. A compound identified by the identification method according to claim 26 or 27.

29. A kit containing at least one member selected from the group consisting of caspase-1, a polynucleotide encoding caspase-1, and a vector containing a polynucleotide encoding caspase-1; and at least one member selected from the group consisting of hepatitis B virus x interacting protein (XIP), a polynucleotide encoding XIP, and a vector containing a polynucleotide encoding XIP.


SEQUENCE LISTING

<110>  DAIICHI PHARMACEUTICAL CO., LTD.

<120>  Degradation inhibitor for Hepatitis B virus x interacting protein

<130>  CLS-002-EP

<150>  JP P2003-045711
<151>  2003-02-24

<160>  25

<170>  PatentIn version 3.1

<210>  1
<211>  91
<212>  PRT
<213>  homo sapiens

<400>  1

Met Glu Ala Thr Leu Glu Gln His Leu Glu Asp Thr Met Lys Asn Pro
1               5                   10                  15

Ser Ile Val Gly Val Leu Cys Thr Asp Ser Gln Gly Leu Asn Leu Gly
            20                  25                  30

Cys Arg Gly Thr Leu Ser Asp Glu His Ala Gly Val Ile Ser Val Leu
        35                  40                  45

Ala Gln Gln Ala Ala Lys Leu Thr Ser Asp Pro Thr Asp Ile Pro Val
    50                  55                  60

Val Cys Leu Glu Ser Asp Asn Gly Asn Ile Met Ile Gln Lys His Asp
65                  70                  75                  80

Gly Ile Thr Val Ala Val His Lys Met Ala Ser
                85                  90

<210>  2
<211>  5
<212>  PRT
<213>  Artificial

<220>
<223>  Partial sequence of Hepatitis B virus x interacting protein (SEQ
       ID NO: 1), which is recognized by caspase-1

<400>  2

His Leu Glu Asp Thr
1               5

<210>  3
<211>  5
<212>  PRT
<213>  Artificial

<220>
<223>  Partial sequence of Hepatitis B virus x interacting protein (SEQ
       ID NO: 1), which is recognized by caspase-1

<400>  3

Leu Cys Thr Asp Ser
1               5

<210>  4
<211>  5
<212>  PRT
<213>  Artificial

<220>
<223>  Partial sequence of Hepatitis B virus x interacting protein (SEQ
       ID NO: 1), which is recognized by caspase-1

<400>  4

Thr Leu Ser Asp Glu
1               5


<210>  5
<211>  8
<212>  PRT
<213>  Artificial

<220>
<223>  Partial sequence of Hepatitis B virus x interacting protein (SEQ
       ID NO: 1), which is recognized by caspase-1

<400>  5

Leu Thr Ser Asp Pro Thr Asp Ile
1               5


<210>  6
<211>  5
<212>  PRT
<213>  Artificial

<220>
<223>  Partial sequence of Hepatitis B virus x interacting protein (SEQ
       ID NO: 1), which is recognized by caspase-1

<400>  6

Leu Glu Ser Asp Asn
1               5


<210>  7
<211>  5
<212>  PRT
<213>  Artificial

<220>
<223>  Partial sequence of Hepatitis B virus x interacting protein (SEQ
       ID NO: 1), which is recognized by caspase-1

<400>  7

Gln Lys His Asp Gly
1               5


<210>  8
<211>  5
<212>  PRT
<213>  Artificial

<220>
<223>  Partial sequence of ProInterleukin-1 beta, which is recognized by
        caspase-1

<400>  8

Tyr Val His Asp Ala
1               5


<210>  9

<211>  5
<212>  PRT
<213>  Artificial

<220>
<223>  Partial sequence of ProInterleukin-18, which is recognized by cas
       pase-1

<400>  9

Leu Glu Ser Asp Asn
1               5


<210>  10
<211>  5
<212>  PRT
<213>  Artificial

<220>
<223>  Partial sequence of Calpastatin, which is recognized by caspase-1

<400>  10

Ala Leu Asp Asp Leu
1               5


<210>  11
<211>  5
<212>  PRT
<213>  Artificial

<220>
<223>  Partial sequence of Calpastatin, which is recognized by caspase-1

<400>  11

Leu Ser Ser Asp Phe
1               5


<210>  12
<211>  5
<212>  PRT
<213>  Artificial

<220>
<223>  Partial sequence of Calpastatin, which is recognized by caspase-1

<400>  12

Ala Leu Ala Asp Ser
1               5


<210>  13
<211>  5
<212>  PRT
<213>  Artificial

<220>
<223>  Partial sequence of PITSLRE kinase, which is recognized by caspas
       e-1

<400>  13

Tyr Val Pro Asp Ser
1               5


<210>  14
<211>  5
<212>  PRT
<213>  Artificial

<220>
<223>  Partial sequence of Caspase-3, which is recognized by caspase-1

<400>  14

Ile Glu Thr Asp Ser
1               5


<210>  15
<211>  5
<212>  PRT
<213>  Artificial

<220>
<223>  Partial sequence of Actin, which is recognized by caspase-1

<400>  15

Leu Val Cys Asp Asn
1               5


<210>  16
<211>  5
<212>  PRT
<213>  Artificial

<220>
<223>  Partial sequence of Actin, which is recognized by caspase-1

<400>  16

Glu Leu Pro Asp Gly
1               5


<210>  17
<211>  5
<212>  PRT
<213>  homo sapiens

<220>
<221>  misc_feature
<223>  Partial sequence of caspase-1 showing high score in the local ali
       gnment between caspase-1 and Hepatitis B virus x interacting prot
       ein


<400>  17

Asp Ser Gln Gly Val
1               5


<210>  18
<211>  5
<212>  PRT
<213>  homo sapiens

<220>
<221>  misc_feature
<223>  Partial sequence of Hepatitis B virus x interacting protein showi
       ng high score in the local alignment between caspase-1 and Hepati
       tis B virus x interacting protein


<400>  18

Asp Ser Gln Gly Leu
1               5


<210>  19
<211>  4
<212>  PRT

<213>  homo sapiens

<220>
<221>  misc_feature
<223>  Partial sequence identical in the sequences of caspase-1 and Hepa
      titis B virus x interacting protein


<400>  19

Asp Ser Gln Gly
1


<210>  20
<211>  15
<212>  PRT
<213>  homo sapiens

<220>
<221>  misc_feature
<223>  Partial sequence of caspase-1 showing high score in the local ali
      gnment between caspase-1 and Hepatitis B virus x interacting prot
      ein


<400>  20

Thr Thr Glu Glu Phe Glu Asp Asp Ala Ile Lys Lys Ala His Ile
1               5                   10                  15


<210>  21
<211>  15
<212>  PRT
<213>  homo sapiens

<220>
<221>  misc_feature
<223>  Partial sequence of Hepatitis B virus x interacting protein showi
      ng high score in the local alignment between caspase-1 and Hepati
      tis B virus x interacting protein


<400>  21

Thr Leu Glu Gln His Leu Glu Asp Thr Met Lys Asn Pro Ser Ile
1               5                   10                  15


<210>  22
<211>  12
<212>  PRT
<213>  homo sapiens

<220>
<221>  misc_feature
<223>  Partial sequence of caspase-1 showing high score in the local ali
      gnment between caspase-1 and Hepatitis B virus x interacting prot
      ein


<400>  22

Ala His Arg Pro Glu His Lys Thr Ser Asp Ser Thr
1               5                   10


<210>  23
<211>  12
<212>  PRT
<213>  homo sapiens

<220>
<221>  misc_feature
<223>  Partial sequence of Hepatitis B virus x interacting protein showi

ng high score in the local alignment between caspase-1 and Hepati
tis B virus x interacting protein

<400> 23

Ala Gln Gln Ala Ala Lys Leu Thr Ser Asp Pro Thr
1               5                   10

<210> 24
<211> 17
<212> PRT
<213> homo sapiens

<220>
<221> misc_feature
<223> Partial sequence of caspase-1 showing high score in the local ali
gnment between caspase-1 and Hepatitis B virus x interacting prot
ein

<400> 24

Gly Met Thr Met Leu Leu Gln Asn Leu Gly Tyr Ser Val Asp Val Lys
1               5                   10                  15

Lys

<210> 25
<211> 17
<212> PRT
<213> homo sapiens

<220>
<221> misc_feature
<223> Partial sequence of Hepatitis B virus x interacting protein showi
ng high score in the local alignment between caspase-1 and Hepati
tis B virus x interacting protein

<400> 25

Gly Asn Ile Met Ile Gln Lys His Asp Gly Ile Thr Val Ala Val His
1               5                   10                  15

Lys